(19) Europäisches Patentamt
European Patent Office
Office européen des brevets

(11) **EP 3 694 394 B1**

(12) **EUROPEAN PATENT SPECIFICATION**

(45) Date of publication and mention
of the grant of the patent:
**05.03.2025 Bulletin 2025/10**

(51) International Patent Classification (IPC):
*A61B 5/00* (2006.01)    *A61B 5/08* (2006.01)
*A61B 5/05* (2021.01)    *A61M 16/01* (2006.01)
*G01N 27/62* (2021.01)

(21) Application number: **18779680.0**

(22) Date of filing: **02.10.2018**

(52) Cooperative Patent Classification (CPC):
**A61B 5/4821; A61B 5/05; A61B 5/082;**
**A61B 5/7203; A61M 16/085;** A61B 5/4845;
A61B 5/7239; A61B 5/7246; A61B 2562/02;
A61M 16/04; A61M 2202/0241; A61M 2205/3317;
A61M 2230/437

(86) International application number:
**PCT/EP2018/076704**

(87) International publication number:
**WO 2019/072620 (18.04.2019 Gazette 2019/16)**

(54) **SYSTEM AND METHOD FOR MEASURING A SUBSTANCE CONCENTRATION IN THE EXHALED BREATH OF A PATIENT**

SYSTEM UND VERFAHREN ZUM MESSEN EINER SUBSTANZKONZENTRATION IN DER AUSATEMLUFT EINES PATIENTEN

SYSTÈME ET PROCÉDÉ POUR MESURER LA CONCENTRATION D'UNE SUBSTANCE DANS L'HALEINE D'UN PATIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.10.2017 EP 17382675**

(43) Date of publication of application:
**19.08.2020 Bulletin 2020/34**

(73) Proprietor: **QUANTIUM MEDICAL S.L.**
**08302 Mataró (Barcelona) (ES)**

(72) Inventors:
• **LINDNER, Claus**
**08005 Barcelona (ES)**
• **GONZÁLES PIJUAN, Carmen**
**08006 Barcelona (ES)**
• **JENSEN, Erik, Weber**
**08395 Sant Pol de Mar (ES)**

(74) Representative: **Fresenius Kabi Deutschland GmbH**
**Patent Department - MedTech**
**Else-Kröner-Straße 1**
**61352 Bad Homburg (DE)**

(56) References cited:
**US-A- 5 455 417    US-A1- 2006 255 258**

• **WEIXIANG ZHAO ET AL: "Gas Chromatography Data Classification Based on Complex Coefficients of an Autoregressive Model", JOURNAL OF SENSORS, vol. 2008, 1 January 2008 (2008-01-01), US, pages 1 - 8, XP055462967, ISSN: 1687-725X, DOI: 10.1155/2008/262501**
• **V RUZSANYI: "Ion mobility spectrometry for pharmacokinetic studies–exemplary application", JOURNAL OF BREATH RESEARCH, vol. 7, no. 4, 28 November 2013 (2013-11-28), US, pages 046008, XP055463076, ISSN: 1752-7155, DOI: 10.1088/1752-7155/7/4/046008**

• PERL T ET AL: "Determination of serum propofol concentrations by breath analysis using ion mobility spectrometry", BRITISH JOURNAL OF ANAESTHESIA, BJM PUBLISHING GROUP, LONDON, GB, vol. 103, no. 6, 1 December 2009 (2009-12-01), pages 822 - 827, XP008128967, ISSN: 0007-0912, [retrieved on 20091103], DOI: 10.1093/BJA/AEP312

**Description**

The invention relates to a system for measuring a substance concentration in the exhaled breath of a patient according to claim 1 and to a method for measuring a substance concentration in the exhaled breath of a patient according to claim 9.

A system of this kind comprises a measurement apparatus for performing an ion mobility spectrometry measurement of a gas probe of the exhaled breath of the patient to obtain a recorded data set indicative of a drift spectrum relating to the gas probe, and a processor for processing the recorded data set to determine at least one characteristic value relating to the drift spectrum and to output a concentration estimate indicative of the substance concentration in the gas probe. Such a system is known from US 2006/255258 A1.

Mechanical ventilation is used in conventional general anesthesia procedures for example in an operating center or during long-term sedation procedures for critically ill patients in an intensive care unit of a hospital. In the context of such general anesthesia procedures patients are intubated with endo-tracheal catheters to on the one hand provide for a ventilation and on the other hand administer gaseous anesthetic agents.

As an alternative to inhalational anesthesia procedures using gaseous anesthetic agents, in the context of intravenous anesthesia an anesthetic agent such as Propofol is administered intravenously into a patient, for example in the context of a so-called total intravenous anesthesia (TIVA) procedure. Such intravenous anesthesia may also be preferable for example for a long-term sedation procedure in an intensive care unit.

In particular in the context of intravenous anesthesia for example using Propofol as an anesthetic agent, it is of substantial interest to be able to monitor the concentration of the anesthetic agent in the patient's body and its related effects in particular with regard to the anesthetic impact. Generally, conclusions with regard to the drug concentration in the patient's body can be drawn by monitoring the presence and concentration of an anesthetic agent and related substances in the exhaled air of a patient. Using a suitable modeling, for example a pharmacokinetic/pharmacodynamic model, the drug concentration in the patient's body can be predicted from the drug concentration in the exhaled air. Such predictions however require on the one hand precise models and on the other hand a precise measuring of substance concentration in the exhaled air.

It recently has been proposed to use ion mobility spectrometry (IMS) for the detection of a substance, in particular when using an anesthetic agent such as Propofol, in the exhaled breath of a patient for monitoring during an anesthesia procedure. Within ion mobility spectrometry, components of a gas probe are ionized and are injected into a drift chamber. By applying a substantial voltage, for example several hundred volts per centimeter, to the drift chamber, the ionized components are driven towards a detector which is constituted to generate a measurement signal upon arrival of the ionized components. The ionized components encounter an opposing force whilst travelling through the drift chamber, which originates from a drift gas that flows through the same drift chamber, but in an opposing direction, thus effectively presenting an obstacle for the ionized components depending on for example their shape and cross section. Generally, the drift time of the ionized components through the drift chamber depends on the applied voltage, but is also influenced by the temperature and pressure in the drift chamber, the mass of the ionized components, their shape and charge and the like, such that different components exhibit different velocities and hence will travel through the drift chamber in different drift times. Different ionized components hence will arrive at the detector at different times, such that signals separated in time and relating to the different ionized components can be detected at the detector, giving rise to a so-called drift spectrum (signal intensity over drift time) containing peaks relating to different components of the gas probe.

To determine the concentration of a substance of interest in the patient's breath, for example an anesthetic agent, in particular Propofol, a peak in a drift spectrum relating to the substance of interest may be identified, and from the peak's height conclusions may be drawn with respect to the concentration of the substance of interest in the gas probe taken from the exhaled breath of the patient. Generally, using a suitable calibration the peak's height may be related to the concentration of the substance of interest, such that from the peak's height the concentration may be directly inferred.

However, a drift spectrum is subject to noise. In addition, concentration values typically lie in the parts-per-billion (ppb) range such that the detected signals are weak. There hence is a desire to be able to accurately determine characteristics of a drift spectrum in order to reliably draw conclusions with respect to a substance concentration in a gas probe.

It is an object of the instant invention to provide a system and method for measuring a substance concentration in the exhaled breath of a patient which allow for accurate measurements using the ion mobility spectrometry (IMS).

This object is achieved by means of a system comprising the features of claim 1 and a method according to claim 9.

Accordingly, the processor is constituted to fit an autoregressive model to at least a portion of the recorded data set to obtain a fitted data set, wherein the processor is further constituted to determine said at least one characteristic value from the fitted data set.

Hence, an autoregressive model is implemented and fitted to the recorded data set. Generally, an autoregressive model (in short: AR) is capable of predicting a value at a specific instance of time from preceding instances of time, such that by applying the autoregressive model a curve may be fitted to the recorded data set obtained from the ion mobility spectrometry measurement. The fitted curve essentially does not comprise noise, such that the fitting of the autoregressive model to the recorded data set may be used to cancel out noise from the recorded data set. Characteristic values, from which the concentration of a substance of interest in the gas probe of the exhaled breath of the patient reliably may be estimated, may then accurately be determined from the fitted data set, such characteristic values being hardly affected by noise.

An autoregressive model which in principle is suitable for application in the instant context is for example described in US 5,673,210 and a research article by W. Zhao et al. entitled "Gas Chromatography Data Classification Based on Complex Coefficients of an Autoregressive Model", Journal of Sensors, 2008, Article ID 262501.

For example, from the fitted data set one or multiple characteristic values such as a value indicative of the height of a peak of interest of the drift spectrum, a value indicative of an area under a curve of the fitted data set (for example under a drift spectrum (along the drift-time axis) or a curve taken along the so-called retention time axis) in the region of a peak of interest, a value indicative of the full width at half maximum of a peak of interest, a value indicative of a skewness of a peak of interest, a value indicative of an inflection point of a peak of interest, and a value indicative of a slope at a peak of interest may be determined. From such characteristic values conclusions with respect to the concentration of the substance of interest in the gas probe may be drawn, wherein for example in a calibration phase prior to the actual, regular operation of the system a range of heights of a peak of interest of the drift spectrum may be related to a corresponding range of concentration values such that from the height of a peak of interest (indicating the signal intensity of a peak relating to a specific substance) the concentration of the specific substance in the gas probe may be determined.

Conclusions with respect to the actual substance concentration in the gas probe may be drawn by evaluating the characteristic values separately. It however is also conceivable that multiple characteristic values are combined for example using a suitable model, such as a generalized linear model, in particular a quadratic model, or the like, to determine an actual concentration value from a multiplicity of characteristic values in combination.

The autoregressive model may for example, in one embodiment, be defined by the equation

$$y_t = \varepsilon_0 + \sum_{i=1}^{M} \varepsilon_{t-i} \cdot y_{t-i} + \xi$$

where M indicates the order of the autoregressive model, $y_t$ indicates the amplitude value of the fitted data set at

time t, $\varepsilon$ are regression coefficients, and $\xi$ is a noise term.

When fitting the autoregressive model to the recorded data set, the regression coefficients are determined for example in an iterative fashion until at least a local or possibly a global minimum is reached. The difference between the fitted data set hence obtained and the recorded data set can be assumed to be predominantly due to noise, such that it can be assumed that noise in the fitted data set is substantially cancelled out. By iteratively fitting the autoregressive model to the recorded data set a substantially noise-free version of the drift spectrum may thus be obtained, allowing for an analysis of the drift spectrum with regard to its characteristic features with an increased accuracy, in particular due to a substantially increased signal-to-noise ratio.

The order of the autoregressive model may for example lie in the range between 10 and 100, for example in between 30 and 70, for example between 40 and 60.

Generally, the autoregressive model may be fitted to the entire recorded data set, i.e., the entire drift spectrum recorded during an ion mobility spectrum measurement. It however is also possible that an autoregressive model is fitted only to a portion of the recorded data set, in particular a subset of the recorded data set relating to a peak of interest of the drift spectrum.

In one embodiment, a one-dimensional autoregressive model is used, which predicts values of a drift spectrum at a specific time from preceding values. In a further embodiment, a two-dimensional autoregressive model is used which, as an additional dimension, takes into account the time variation between different drift spectra.

Generally, multiple drift spectra relating to a gas probe may be determined using ion mobility spectrometry, the multiple drift spectra indicating the signal variation over time. Hence, multiple recorded data sets may be obtained, each recorded data set relating to a drift spectrum at a specific instance of time, the multiple recorded data sets hence indicating the variation of the drift spectra over time, the time between two consecutively acquired drift spectra also being referred to as retention time.

The second dimension of the autoregressive model may be the retention time axis. The fit of the autoregressive model to the recorded data sets hence does not only take place along the drift time axis, but also along the retention time axis, which may additionally improve accuracy and may give rise to additional characteristic values relating in particular to the time variation of peaks in recorded drift spectra.

By means of the system, characteristic values relating to one or multiple drift spectra may be determined, which may then be used, using a suitable calibration, to output a concentration estimate indicative of substance concentrations in the gas probe. The substance(s) of interest may in particular be at least one anesthetic agent, for example Propofol, such that the system may in particular be suitable for providing a monitoring during an anesthesia or sedation procedure, for example an anesthesia procedure in which an anesthetic agent is intravenously administered to a patient and the concentration of the anesthetic agent in the patient's body is monitored using ion mobility spectrometry on a gas probe of the exhaled breath of the patient.

For this, gas probes may be taken continuously or periodically in order to monitor the concentration(s) of at least one anesthetic agent, in particular Propofol, in the exhaled breath of a patient.

The object is also achieved by a method for measuring a substance concentration in the exhaled breath of a patient, the method comprising: performing an ion mobility spectrometry measurement of a gas probe of the exhaled breath of the patient, using a measurement apparatus, to obtain a recorded data set indicative of a drift spectrum relating to the gas probe, and processing the recorded data set, using a processor, to determine at least one characteristic value relating to the drift spectrum and to output a concentration estimate indicative of the substance concentration in the gas probe. Herein, said processing includes fitting an autoregressive model to the recorded data set to obtain a fitted data set, and determining said at least one characteristic value from the fitted data set.

The advantages and advantageous embodiments described above for the system equally apply also to the method, such that it shall be referred to the above.

The idea underlying the invention shall subsequently be described in more detail with reference to the embodiments shown in the drawings. Herein:

Fig. 1    shows a schematic view of a system comprising an endo-tracheal catheter and a ventilation system for providing ventilation to a patient;

Fig. 2    shows a graphical representation of a drift spectrum recorded by using ion mobility spectrometry on a gas probe taken from the exhaled breath of a patient;

Fig. 3    shows a portion of a drift spectrum in the range of a peak relating to Propofol (top graph) and the difference between recorded data and fitted data (bottom graph); and

Fig. 4    shows a flow chart of a workflow for determining a concentration estimate from a recorded data set obtained using ion mobility spectrometry.

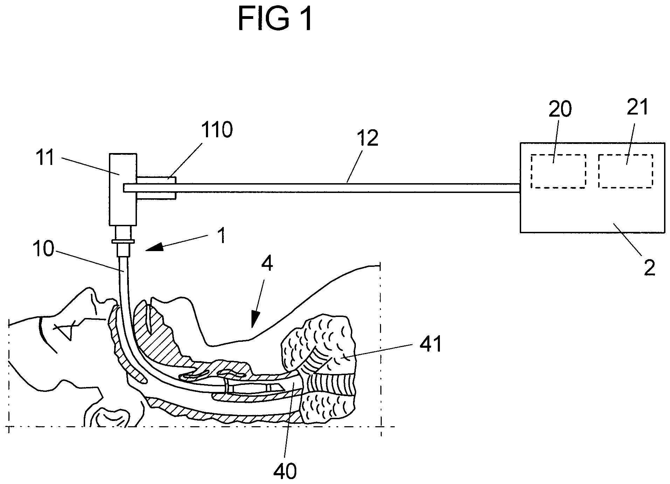

Fig. 1 shows an embodiment of a system as it generally may be used for example in the context of an anesthesia procedure.

For example in an intravenous anesthesia procedure, an anesthetic agent such as Propofol is intravenously administered to a patient 4 and hence enters into the patient's bloodstream. In order to monitor the concentration of the anesthetic substance within the patient's body, a gas detector 2 connected in a sidestream arrangement to a connection piece 11 of an endo-tracheal catheter 1 continuously or periodically measures a drug concentration in a gaseous flow taken from the patient's lungs 41 via a catheter tube 10 of the endo-tracheal catheter 1 inserted into the trachea 40 of the patient 4. By means of such concentration measurements, hence, a monitoring of the substance concentration in the exhaled air of the patient 4 may be conducted, allowing for conclusions with respect to the concentration of the anesthetic substance within the patient's body, for example using a suitable pharmacokinetic/pharmacodynamic model or the like.

In an embodiment, the gas detector 2 comprises a processor 20 and a measurement apparatus 21, the measurement apparatus 21 being designed to conduct ion mobility spectrometry measurements on gas probes taken via a side-stream line 12 connected at a port 110 to the connection piece 11 of the endo-tracheal catheter 1.

By means of the side-stream line 12 gas probes may be taken in a continuous or periodic fashion from a gas flow streaming through the connection piece 11. In order to measure the concentration of a substance of interest in a gas probe taken from the exhaled breath of the patient 4, ion mobility spectrometry is used in which components of the gas probe are ionized and injected into a drift chamber, through which the components are driven by a substantial voltage, for example larger than 100 volt or even a few hundred volt per centimeter of drift, towards a detector. By means of the detector a measurement signal is obtained, generated by the ionized components arriving at the detector and causing a low voltage signal at the detector. Because different components exhibit different drift velocities through the drift chamber - for example dependent on the temperature and pressure in the drift chamber, the component's mass, and the component's shape and charge - different components will arrive at the detector at different drift times, causing a drift spectrum in which peaks occur relating to the different drift times of the different components. From the signal intensity at a peak relating to a specific component relating to a substance of interest, hence, conclusions can be drawn with regard to the concentration of the substance of interest in the gas probe taken from the gas flow.

In order to draw conclusions with regard to the substance concentration in the gas probe, characteristic values in relation to a drift spectrum are determined, such characteristic values allowing to infer a concentration estimate (by for example calibrating, in an initial calibration phase, a range of a characteristic value to a range of concentration values). Recorded data obtained from ion mobility spectrometry however generally is subject to noise, such that it may not be possible, without further ado, to accurately determine characteristic values such as the height of a peak relating to a substance of interest in a drift spectrum with sufficient accuracy.

For this it is proposed to use an autoregressive model defined by the equation

$$y_t = \varepsilon_0 + \sum_{i=1}^{M} \varepsilon_{t-i} \cdot y_{t-i} + \xi,$$

where M indicates the order of the autoregressive model, $y_t$ indicates the amplitude value of the fitted data set at time t, $\varepsilon$ are regression coefficients, and $\xi$ is a noise term. The autoregressive model may iteratively be fitted to the recorded data obtained from ion mobility spectrometry, such that iteratively the regression coefficients of the autoregressive model are determined until a reasonable fit of the autoregressive model to the recorded data is reached, for example when a local minimum in the iterative fitting procedure is reached. By fitting the autoregressive model to the recorded data, fitted data is obtained, wherein the difference between the fitted data and the recorded data can be assumed to substantially be due to noise, the fitted data hence essentially representing a noise-reduced version of the drift spectrum of the recorded data. From the fitted data, hence, characteristic values of the drift spectrum may be determined with increased accuracy, because the fitted data generally exhibits a large signal-to-noise ratio.

This makes it possible to determine a variety of different characteristic values with sufficient accuracy such that conclusions with respect to the concentration of the substance of interest in the gas probe can be drawn. For example, the height of one or multiple peaks of interest relating to one or multiple substances of interest, an area under the curve of the drift spectrum at a peak of interest, the full width at half maximum at a peak of interest, the peak skewness, a peak's inflection points, slope values or the like may be determined. In addition, for example a derivative of a drift spectrum may be determined and processed. Furthermore, conclusions may be drawn from the regression coefficients as such.

Such characteristic values may be used separately to determine a concentration estimate. For example, from a peak's height, which directly relates to the signal intensity of a substance of interest, a concentration estimate may be inferred.

In addition or alternatively, it is possible to combine several characteristic values for example in a suitable model in order to determine a concentration estimate from a combination of different characteristic values.

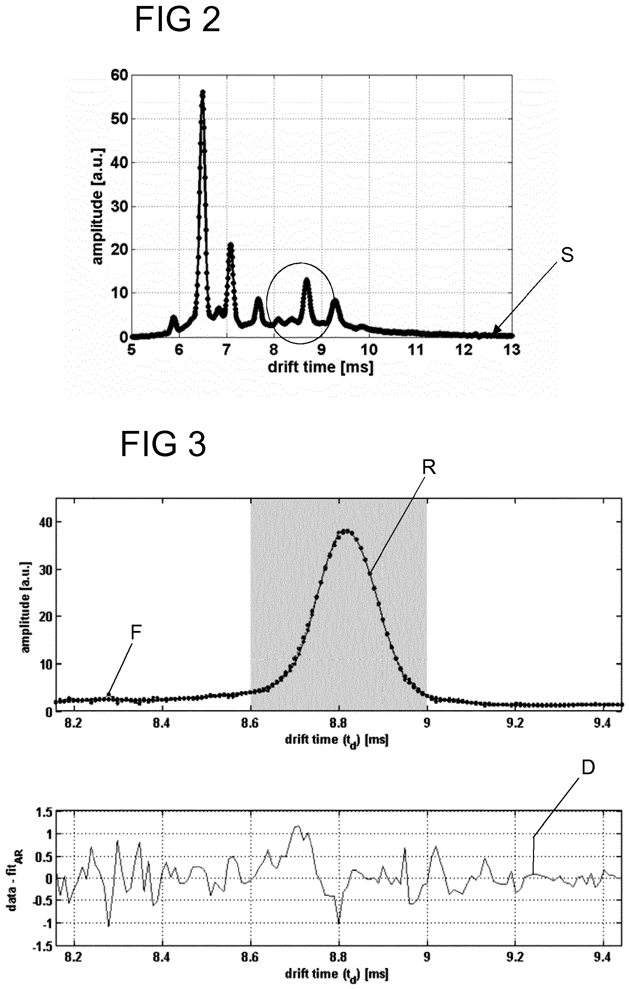

Fig. 2 shows an example of a recorded drift spectrum S obtained by an ion mobility spectrometry measurement using the measurement apparatus 21 of the gas detector 2. As it is visible, the drift spectrum S contains various peaks, the different peaks relating to different components of the gas probe.

Fig. 3 relates to a portion of a drift spectrum S, showing - in the top graph - a recorded data set R (represented by the dots in the top graph) in the range of a peak of interest and a fitted data set F (represented by the continuous line in the top graph) which is obtained by fitting the autoregressive model to the recorded data set R and - in the bottom graph - the difference D between the recorded data set R and the fitted data set F. (The increased discrepancies between the recorded data R and the fitted data F in the first portion of the drift time window are due to the nature of the autoregressive model and the chosen data excerpt and can be significantly reduced by selecting a broader window including more data points.)

The autoregressive model indicated by the above equation is one-dimensional in that a value at a specific time depends on previous values along the drift time axis. In addition, a second dimension may be introduced taking into account the so-called retention time axis.

Specifically, multiple drift spectra S may be recorded over a period of time in order to take into account the variation of the drift spectra over time. Multiple recorded data sets R relating to multiple drift spectra S hence are obtained, to which the autoregressive model may be fitted using the retention time axis as an additional dimension for the model (the retention time denotes the time between instances of consecutively recorded drift spectra).

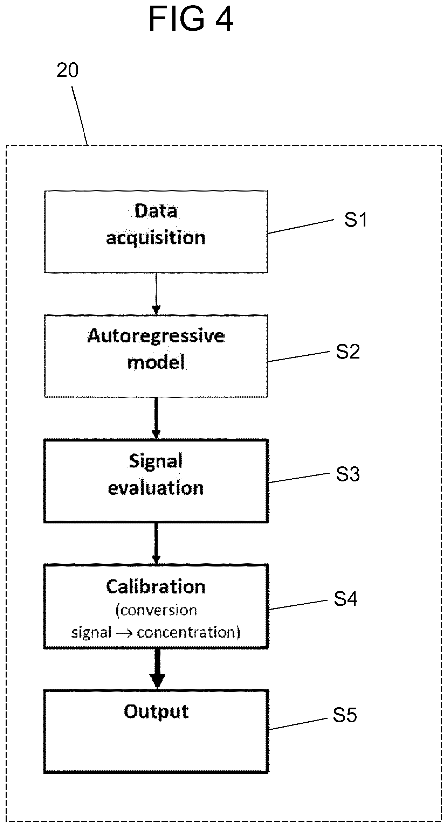

Fig. 4 illustrates a workflow for processing recorded data sets obtained using ion mobility spectrometry for analyzing gas probes of a gas flow for example during an anesthesia procedure. For the processing of data, in a first step S1 data is acquired to obtain one or multiple recorded data sets R. To the recorded data sets R an autoregressive model is fitted in a step S2 to obtain one or multiple fitted data sets F, and in a step S3 one or multiple drift spectra S obtained according to the fitted data sets F are evaluated to determine one or multiple characteristic values of the drift spectra S. According to a calibration the one or the multiple characteristic values are converted into a concentration estimate in a step S4, which in a step S5 is then the output.

The idea underlying the invention is not limited to the embodiments described and is defined in the claims.

In particular, the approach as described herein may not only be used within the context of an anesthesia procedure, but may generally be used to monitor a substance of interest in the exhaled breath of a patient using ion mobility spectrometry.

**List of Reference Numerals**

| | |
|---|---|
| 1 | Endo-tracheal catheter |
| 10 | Catheter tube |
| 11 | Connection piece |
| 110 | Port |

| 12 | Side-stream line |
|---|---|
| 2 | Detection device |
| 20 | Processor |
| 21 | Measurement apparatus |
| 3 | Ventilation system |
| 30 | Ventilator |
| 31 | Filter |
| 32 | Ventilation lines |
| 4 | Patient |
| 40 | Trachea |
| 41 | lungs |
| D | Difference |
| F | Fitted data set |
| R | Recorded data set |
| S | Drift spectrum |
| S1-S5 | Steps |

**Claims**

1. System for measuring a substance concentration in the exhaled breath of a patient (4), during an intravenous anesthesia procedure comprising:

   - a measurement apparatus (21) for performing an ion mobility spectrometry measurement of a gas probe of the exhaled breath of the patient to obtain a recorded data set (R) indicative of a drift spectrum (S) relating to the gas probe, and
   - a processor (20) for processing the recorded data set (R) to determine at least one characteristic value relating to the drift spectrum (S) and to output a concentration estimate indicative of the substance concentration in the gas probe,

   **characterized in**

   **that** the processor (20) is constituted to fit an autoregressive model to at least a portion of the recorded data set (R) to obtain a fitted data set (F), wherein the processor (20) is further constituted to determine said at least one characteristic value from the fitted data set (F), wherein the autoregressive model is defined by the equation

   $$y_t = \varepsilon_0 + \sum_{i=1}^{M} \varepsilon_{t-i} \cdot y_{t-i} + \xi$$

   where M indicates the order of the autoregressive model, $y_t$ indicates the amplitude value of the fitted data set at time $t$, $\varepsilon$ are regression coefficients, and $\xi$ is a noise term, and the order M lies in a range between 10 and 100, preferable in a range between 30 and 70, for example between 40 and 60.

2. System according to claim 1, **characterized in that** the at least one characteristic value includes at least one of a value indicative of the height of a peak of interest of the drift spectrum (S), a value indicative of an area under a curve obtained from the fitted data set (F) in the region of a peak of interest, a value indicative of the full width at half maximum of a peak of interest, a value indicative of a skewness of a peak of interest, a value indicative of an inflection point of a peak of interest, and a value indicative of a slope at a peak of interest.

3. System according to one of the preceding claims, **characterized in that** the processor (20) is constituted to determine, during the fitting, the regression coefficients $\varepsilon$ of the autoregressive model.

4. System according to one of the preceding claims, **characterized in that** the processor (20) is constituted to fit the autoregressive model to at least a subset of the recorded data set (R) in the range of a peak of interest of the drift spectrum (S).

5. System according to one of the preceding claims, **characterized in that** the processor (20) is constituted to apply the autoregressive model in either one or two dimensions.

6. System according to one of the preceding claims, **characterized in that** the measurement apparatus (21) is constituted to obtain multiple recorded data sets (R) indicative of multiple time-varying drift spectra (S).

7. System according to claim 6, **characterized in that** the processor (20) is constituted to fit the autoregressive model to the multiple recorded data sets (R) to obtain multiple fitted data sets (F).

8. System according to one of the preceding claims, **characterized in that** the concentration estimate is indicative of the concentration of an anesthetic agent, in particular Propofol, in the gas probe.

9. Method for measuring a substance concentration in the exhaled breath of a patient (4), during an intravenous anesthesia procedure comprising:

   - performing an ion mobility spectrometry measurement of a gas probe of the exhaled breath of the patient, using a measurement apparatus (21), to obtain a recorded data set (R) indicative of a drift spectrum (S) relating to the gas probe, and
   - processing the recorded data set (R), using a processor (20), to determine at least one characteristic value relating to the drift spectrum (S) and to output a concentration estimate indicative of the substance concentration in the gas probe,

**characterized in**

**that** said processing includes fitting an autoregressive model to the recorded data set (R) to obtain a fitted data set (F), and determining said at least one characteristic value from the fitted data set (F), wherein the autoregressive model is defined by the equation

$$y_t = \varepsilon_0 + \sum_{i=1}^{M} \varepsilon_{t-i} \cdot y_{t-i} + \xi$$

where M indicates the order of the autoregressive model, $y_t$ indicates the amplitude value of the fitted data set at time $t$, $\varepsilon$ are regression coefficients, and $\xi$ is a noise term, and the order M lies in a range between 10 and 100, preferable in a range between 30 and 70, for example between 40 and 60.

**Patentansprüche**

1. System zum Messen einer Substanzkonzentration in der Ausatemluft eines Patienten (4) während einer Prozedur mit intravenöser Narkose, umfassend:

   - ein Messgerät (21) zum Durchführen einer Ionenmobilitätsspektrometrie-Messung einer Gassonde der Ausatemluft des Patienten, um einen aufgezeichneten Datensatz (R) zu erhalten, der ein Driftspektrum (S) in Bezug auf die Gassonde angibt, und
   - einen Prozessor (20) zum Verarbeiten des aufgezeichneten Datensatzes (R) zum Bestimmen mindestens eines charakteristischen Werts, der sich auf das Driftspektrum (S) bezieht, und zum Ausgeben einer Konzentrationsschätzung, die die Substanzkonzentration in der Gassonde angibt, **dadurch gekennzeichnet,**

   **dass** der Prozessor (20) so aufgebaut ist, dass er ein autoregressives Modell an mindestens einen Teil des aufgezeichneten Datensatzes (R) anpasst, um einen angepassten Datensatz (F) zu erhalten, wobei der Prozessor (20) ferner so aufgebaut ist, dass er den mindestens einen charakteristischen Wert aus dem angepassten Datensatz (F) bestimmt, wobei das autoregressive Modell durch folgende Gleichung definiert ist

$$y_t = \varepsilon_0 + \sum_{i=1}^{M} \varepsilon_{t-i} \cdot y_{t-i} + \xi$$

wobei M die Ordnung des autoregressiven Modells angibt, $y_t$ den Amplitudenwert des angepassten Datensatzes zum Zeitpunkt t angibt, $\varepsilon$ Regressionskoeffizienten sind und $\xi$ ein Rauschterm ist und die Ordnung M in einem Bereich zwischen 10 und 100, vorzugsweise in einem Bereich zwischen 30 und 70, beispielsweise zwischen 40 und 60 liegt.

2. System nach Anspruch 1, **dadurch gekennzeichnet, dass** der mindestens eine charakteristische Wert mindestens einen von einem Wert, der die Höhe eines Peaks von Interesse des Driftspektrums (S) angibt, einem Wert, der eine Fläche unter einer Kurve angibt, die aus dem angepassten Datensatz (F) in der Region eines Peaks von Interesse erhalten wird, einem Wert, der die volle Breite bei halbem Maximum eines Peaks von Interesse angibt, einem Wert, der eine Schiefe eines Peaks von Interesse angibt, einem Wert, der einen Wendepunkt eines Peaks von Interesse angibt, und einem Wert, der eine Steigung an einem Peak von Interesse angibt, beinhaltet.

3. System nach einem der vorhergehenden Ansprüche, **dadurch gekennzeichnet, dass** der Prozessor (20) so aufgebaut ist, dass er während der Anpassung die Regressionskoeffizienten $\varepsilon$ des autoregressiven Modells bestimmt.

4. System nach einem der vorhergehenden Ansprüche, **dadurch gekennzeichnet, dass** der Prozessor (20) so aufgebaut ist, dass er das autoregressive Modell an mindestens eine Teilmenge des aufgezeichneten Datensatzes (R) im Bereich eines Peaks von Interesse des Driftspektrums (S) anpasst.

5. System nach einem der vorhergehenden Ansprüche, **dadurch gekennzeichnet, dass** der Prozessor (20) so aufgebaut ist, dass er das autoregressive Modell in einer oder zwei Dimensionen anwendet.

6. System nach einem der vorhergehenden Ansprüche, **dadurch gekennzeichnet, dass** das Messgerät (21) so aufgebaut ist, dass es mehrere aufgezeichnete Datensätze (R) erhält, die mehrere zeitveränderliche Driftspektren (S) angeben.

7. System nach Anspruch 6, **dadurch gekennzeichnet dass** der Prozessor (20) so aufgebaut ist, dass er das autoregressive Modell an die mehreren aufgezeichneten Datensätze (R) anpasst, um mehrere angepasste Datensätze (F) zu erhalten.

8. System nach einem der vorhergehenden Ansprüche, **dadurch gekennzeichnet, dass** die Konzentrationsschätzung die Konzentration eines Anästhetikums, insbesondere Propofol, in der Gassonde angibt.

9. Verfahren zum Messen einer Substanzkonzentration in der Ausatemluft eines Patienten (4) während einer Prozedur mit intravenöser Narkose, umfassend:

- Durchführen einer Ionenmobilitätsspektrometrie-Messung einer Gassonde der Ausatemluft des Patienten, unter Verwendung eines Messgerätes (21), um einen aufgezeichneten Datensatz (R) zu erhalten, der ein Driftspektrum (S) in Bezug auf die Gassonde angibt, und
- Verarbeiten des aufgezeichneten Datensatzes (R) unter Verwendung eines Prozessors (20), um mindestens einen charakteristischen Wert in Bezug auf das Driftspektrum (S) zu bestimmen und eine Konzentrationsschätzung auszugeben, die die Substanzkonzentration in der Gassonde angibt, **dadurch gekennzeichnet,**

**dass** die Verarbeitung das Anpassen eines autoregressiven Modells an den aufgezeichneten Datensatz (R), um einen angepassten Datensatz (F) zu erhalten, und das Bestimmen des mindestens einen charakteristischen Wertes aus dem angepassten Datensatz (F) beinhaltet, wobei das autoregressive Modell durch folgende Gleichung definiert ist

$$y_t = \varepsilon_0 + \sum_{i=1}^{M} \varepsilon_{t-i} \cdot y_{t-i} + \xi$$

wobei M die Ordnung des autoregressiven Modells angibt, $y_t$ den Amplitudenwert des angepassten Datensatzes zum Zeitpunkt t angibt, $\varepsilon$ Regressionskoeffizienten sind und $\xi$ ein Rauschterm ist und die Ordnung M in einem Bereich zwischen 10 und 100, vorzugsweise in einem Bereich zwischen 30 und 70, beispielsweise zwischen 40 und 60 liegt.

**Revendications**

1. Système de mesure d'une concentration de substance dans l'air expiré d'un patient (4), au cours d'une procédure d'anesthésie intraveineuse comprenant :

- un appareil de mesure (21) permettant d'effectuer une mesure par spectrométrie de mobilité ionique d'une sonde à gaz de l'air expiré du patient pour obtenir un ensemble de données enregistrées (R) indiquant un spectre de dérive (S) relatif à la sonde à gaz, et
- un processeur (20) permettant de traiter l'ensemble de données enregistrées (R) pour déterminer au moins une valeur caractéristique relative au spectre de dérive (S) et pour produire une estimation de concentration indiquant la concentration de substance dans la sonde à gaz, **caractérisé en**

**ce que** le processeur (20) est constitué pour ajuster un modèle autorégressif à au moins une partie de l'ensemble de données enregistrées (R) pour obtenir un ensemble de données ajustées (F), dans lequel le processeur (20) est en outre constitué pour déterminer ladite au moins une valeur caractéristique à partir de l'ensemble de données ajustées (F), dans lequel le modèle autorégressif est défini par l'équation

$$y_t = \varepsilon_0 + \sum_{i=1}^{M} \varepsilon_{t-i} \cdot y_{t-i} + \xi$$

où M indique l'ordre du modèle autorégressif, $y_t$ indique la valeur d'amplitude de l'ensemble de données ajustées à l'instant t, $\varepsilon$ sont des coefficients de régression, et est un terme de bruit, et l'ordre M se situe dans une fourchette comprise entre 10 et 100, de préférence dans une fourchette comprise entre 30 et 70, par exemple entre 40 et 60.

2. Système selon la revendication 1, **caractérisé en ce que** l'au moins une valeur caractéristique comporte au moins l'une parmi une valeur indiquant la hauteur d'un pic d'intérêt du spectre de dérive (S), une valeur indiquant l'aire sous une courbe obtenue à partir de l'ensemble de données ajustées (F) dans la région d'un pic d'intérêt, une valeur indiquant la largeur totale à mi-maximum d'un pic d'intérêt, une valeur indiquant une asymétrie d'un pic d'intérêt, une valeur indiquant un point d'inflexion d'un pic d'intérêt, et une valeur indiquant une pente au niveau d'un pic d'intérêt.

3. Système selon l'une des revendications précédentes, **caractérisé en ce que** le processeur (20) est constitué pour déterminer, pendant l'ajustement, les coefficients de régression $\varepsilon$ du modèle autorégressif.

4. Système selon l'une des revendications précédentes, **caractérisé en** ce que le processeur (20) est constitué pour ajuster le modèle autorégressif à au moins un sous-ensemble de l'ensemble de données enregistrées (R) dans la fourchette d'un pic d'intérêt du spectre de dérive (S).

**5.** Système selon l'une des revendications précédentes, **caractérisé en ce que** le processeur (20) est constitué pour appliquer le modèle autorégressif en soit une soit deux dimensions.

**6.** Système selon l'une des revendications précédentes, **caractérisé en ce que** l'appareil de mesure (21) est constitué pour obtenir de multiples ensembles de données enregistrées (R) indiquant de multiples spectres de dérive variant dans le temps (S).

**7.** Système selon la revendication 6, **caractérisé en ce que** le processeur (20) est constitué pour ajuster le modèle autorégressif aux multiples ensembles de données enregistrées (R) pour obtenir de multiples ensembles de données ajustées (F).

**8.** Système selon l'une des revendications précédentes, **caractérisé en** ce que l'estimation de concentration indique la concentration d'un agent anesthésique, en particulier le Propofol, dans la sonde à gaz.

**9.** Procédé de mesure d'une concentration de substance dans l'air expiré d'un patient (4), au cours d'une procédure d'anesthésie intraveineuse comprenant :

   - la mise en œuvre d'une mesure par spectrométrie de mobilité ionique d'une sonde de gaz de l'air expiré du patient, à l'aide d'un appareil de mesure (21), pour obtenir un ensemble de données enregistrées (R) indiquant un spectre de dérive (S) relatif à la sonde à gaz, et
   - le traitement de l'ensemble de données enregistrées (R), à l'aide d'un processeur (20), pour déterminer au moins une valeur caractéristique relative au spectre de dérive (S) et pour produire une estimation de concentration indiquant la concentration de substance dans la sonde à gaz, **caractérisé en**

      **ce que** le traitement comporte l'ajustement d'un modèle autorégressif à l'ensemble de données enregistrées (R) pour obtenir un ensemble de données ajustées (F), et la détermination de ladite au moins une valeur caractéristique à partir de l'ensemble de données ajustées (F), dans lequel le modèle autorégressif est défini par l'équation

$$y_t = \varepsilon_0 + \sum_{i=1}^{M} \varepsilon_{t-i} \cdot y_{t-i} + \xi$$

      où M indique l'ordre du modèle autorégressif, $y_t$ indique la valeur d'amplitude de l'ensemble de données ajustées à l'instant t, $\varepsilon$ sont des coefficients de régression, et est un terme de bruit, et l'ordre M se situe dans une fourchette comprise entre 10 et 100, de préférence dans une fourchette comprise entre 30 et 70, par exemple entre 40 et 60.

FIG 1

# FIG 2

# FIG 3

# FIG 4

**EP 3 694 394 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006255258 A1

- US 5673210 A

**Non-patent literature cited in the description**

- **W. ZHAO et al.** Gas Chromatography Data Classification Based on Complex Coefficients of an Autoregressive Model. *Journal of Sensors*, 2008